# EUROPEAN PATENT SPECIFICATION

(11) **EP 0 574 508 B1**
(45) Date of publication and mention of the grant of the patent: **12.11.1997**
(21) Application number: 92907838.4
(22) Date of filing: 14.02.1992
(51) Int. Cl.: C12M 1/34, G01N 33/49, G01N 33/50

(54) **METHOD AND APPARATUS FOR SCREENING MICROSCOPIC CELLS UTILIZING POLARIZED LIGHT SCATTER TECHNIQUES**
VERFAHREN UND VORRICHTUNG ZUM REIHENSICHTEN MIKROSKOPISCHER ZELLEN UNTER VERWENDUNG VON TECHNIKEN ZUR STREUUNG POLARISIERTEN LICHTS
PROCEDE ET APPAREIL POUR DEPISTER DES CELLULES MICROSCOPIQUES A L'AIDE DE TECHNIQUES DE DISPERSION DE LUMIERE POLARISEE

(30) Priority: 22.02.1991 US 644637
(43) Date of publication of application: 22.12.1993
(73) Proprietor: COULTER CORPORATION, Hialeah, FL 33010 (US)
(72) Inventor: HUDSON, James, Carey, Miami Lakes, FL 33014 (US); RODRIGUEZ, Carlos, M., Miami, FL 33165 (US); RUSSELL, Thomas, Miami, FL 33186 (US)
(74) Representative: Perry, Robert Edward
(86) International application number: US9201234
(87) International publication number: WO9214812

(56) References cited:
- WO-A-88/07199
- WO-A-90/13013
- US-A- 4 662 742
- US-A- 4 717 655
- US-A- 4 727 020
- US-A- 4 752 563

## Description

### Technical Field

This invention relates generally to a method and apparatus for screening microscopic cells which express selected characteristics utilizing polarized light scatter techniques. More particularly, the invention is directed to an analysis of one or more cell groups of interest obscured in a cell population by utilizing microspheres having monoclonal antibodies bound thereto to change the sensed characteristics of each specified cell to differentiate the cells of the cell group of interest from the cell population.

### Background Art

This invention relates generally to an automated analyzer and methods of using same for screening biological cells or formed bodies for the enumeration of populations which express selected characteristics for research, diagnostic, medical or industrial purposes. More particularly, the automated analyzers and methods embodying the invention enable multiple part classifications of cells and formed bodies, functional phenotyping of cells and formed bodies, typing of leukemic, lymphoma and solid tumor cells, among others, using a unique combination of optical or optical and electronic technology and the specificity of selective biological molecules, such as antibodies, for such screening and selective enumeration of the cells and formed bodies.

Automation of routine complete blood cell (CBC) analysis of human peripheral blood by an automated blood cell counter was successfully achieved by the COULTER COUNTER (registered trademark) Model A of Coulter Electronics, Inc. of Hialeah, Florida. The electronic particle sensing system principle of that instrument is disclosed in U.S. Patent No. 2,656,508 issued October 20, 1953 to Wallace H. Coulter. This Coulter sensing principle was developed and expanded into more sophisticated instrumentation such as the COULTER COUNTER (registered trademark) Model S types of instruments which enabled CBC parameters, absolute cell counts, platelet count and morphology, red blood cell (RBC) morphology, interpretation of normal and abnormal blood specimens by specific computer programs.

The Coulter electronic particle sensing principle employs an aperture sensing circuit using a direct current (DC) aperture supply. Such particle sensors are simple in structure, extremely rugged and reliable as attested to by the substantially universal acceptance of the COULTER COUNTER (registered trademark) automated analyzer in clinical laboratories in the United States and throughout the rest of the World. An improvement in this basic aperture sensing circuit was disclosed in U.S. Patent No. 3,502,974 issued in 1970 to Wallace Coulter and Walter Hogg. In addition to the standard direct current aperture supply, a high frequency aperture current was applied which enabled the sensing of an additional parameter for classification purposes. The high frequency aperture current produced a signal which is the function of the blood cell's internal conductivity as well as its volume. The signal produced simultaneously by the direct current aperture circuit is a conventional DC amplitude signal which provides an indication primarily of cell volume. The radio frequency amplitude is divided by the direct current pulse amplitude employing a high speed divider circuit to obtain a quotient which is a function of cell volume and internal resistance, conveniently referred to as "opacity". This principle is further described in U.S. Patent No. 3,502,973 also issued to Wallace Coulter and Walter Hogg, in 1970. The opacity parameter has applicability in cell classification systems. Either a single or a pair of separate apertures could be utilized for this purpose.

Classification of different populations is accomplished by collating the data of the signal pairs as they are produced; one, a measure of particle volume and the other a measure of cell internal resistivity or opacity. A convenient form of presenting this data is by two-dimensional plots referred to as scatterplots or scattergrams. Such plots are well described in Flow Cytometry and Sorting, page 371, edited by Melamed Melaney, and Medelsohn, 1979, John Wiley & Sons, NY, NY.

Initial applications of the Coulter electronic particle sensing principle was to perform red blood cell counts and then, more sophisticated determinations of other red blood cell parameters. By removing red blood cells from whole peripheral blood, analysis of the white blood cell (WBC) populations could be undertaken so long as the red blood cell removal did not significantly impair properties of the remaining white blood cell populations sought to be measured. Red blood cell lysing reagents were developed for this purpose which, though useful and widely applied, were not entirely satisfactory in all respects for subsequent white blood cell determinations.

Previous methods of flow analysis of leukocytes using DC volume alone or light scatter at various angles have shown three clusters of leukocytes corresponding to lymphocytes, monocytes and granulocytes which included the neutrophil, basophil and eosinophil populations. A rough but useful estimation of eosinophil concentration can be made on some samples. The fifth major population, basophils, is relatively too small for this approach. The eosinophils also have been observed as a distinct cluster using special fluorescence techniques.

Immunologic studies also are important when anomalies are found on a peripheral blood smear. It is necessary to determine the specific subtype of the leukemia in order to better select a treatment method for the disease and to provide the patient with as specific a prognosis as possible. For example, in forms of acute leukemia, there is a predominance of blasts in the peripheral blood. These immature cells can be difficult to classify as either lymphocytic or granulocytic because of the lack of differentiation. If the blast subpopulation that is rapidly proliferating is found to be T11 receptor bearing, the leukemia can be classified as an acute lymphoblastic leukemia, T-cell type. In general, T lineage ALL has a poorer prognosis, than B lineage ALL. Further subgrouping these leukemias according to their level of differentiation is also customary. Groups I and II exhibit antigens that are seen on early thymic precursor cells; while those expressed in Group III are similar to the surface antigens found on mature T cells. Information such as this regarding the surface antigens expressed on leukemic cells is useful for patient prognosis and treatment.

Immunology experiments were first developed utilizing a light microscope for determination of lymphocyte subsets. Rosette formation between human lymphocytes and sheep red blood cells was observed by Coombs and others in 1970. Later studies found that all or at least a major portion of thymus-derived lymphocytes (T-cells) under the proper conditions displayed the rosette formation phenomenon. These studies utilized Ficoll isolated lymphocytes and were for a period of time routinely employed for subset classification of isolated lymphocytes utilizing a light microscope.

Lymphocyte subsets now conventionally are determined by fluorescent labeling of the cells, in a sample with a fluorescent-tagged monoclonal antibody. The fluorescent-tagged monoclonal antibody binds to the antigen of interest on the surface of the cells expressing the antigen. The cell sample then is analyzed by utilizing a fluorescent microscope or by utilizing a highly sophisticated flow cytometry instrument. When utilizing a flow cytometry instrument, the cell sample preparation, data collection and data analysis must be performed by a specially trained technician. The flow cytometry instrument includes a laser and complex optical system, a high-power computer and electrical and fluidic systems. The component systems of the flow cytometry instrument must be properly maintained and calibrated on a regular and frequent basis. Although the flow cytometry instrument currently is the reference lymphocyte subset determination method, the method has several drawbacks including the high cost of the instrument and the expertise required to correctly operate such instrument.

Lymphocyte subsets also can be determined utilizing automated instruments and methods developed by the assignee of the present application, Coulter Electronics, Inc. An improved simple automated instrument and methods of using the same is disclosed in U.S. Serial No. 587,646, filed September 20, 1990 (US-A-5223398), entitled AUTOMATED ANALYZER AND METHOD FOR SCREENING CELLS OR FORMED BODIES FOR ENUMERATION OF POPULATIONS EXPRESSING SELECTED CHARACTERISTICS which is a continuation of U.S. Serial No. 025,345, filed March 13, 1987 (WO-A-8807199) of the same title. This application combines the application of electronic sensing principles, the specificity of selected biological molecules for identifying and/or enumerating defined populations of cells or formed bodies and microscopic particle technology. The automated analyzer can be used together with a special lysing reagent and/or antibodies coupled to microscopic microspheres or supports of varying composition.

A second application, U.S. Serial No. 285,856, filed December 16, 1988 (WO-A-9007259), entitled METHOD AND APPARATUS FOR SCREENING CELLS OR FORMED BODIES WITH POPULATIONS EXPRESSING SELECTED CHARACTERISTICS, discloses the screening of direct subsets from whole blood samples or portions thereof.

A third application, U.S. Serial No. 339,156, filed April 14, 1989 (WO-A-9013013), entitled METHOD AND APPARATUS FOR SCREENING CELLS OR FORMED BODIES WITH POPULATIONS EXPRESSING SELECTED CHARACTERISTICS UTILIZING AT LEAST ONE SENSING PARAMETER, discloses multipart or five part white blood cell differentials, lymphocyte subsets and overlapping determinations performed from a whole blood sample or from a sample with the red blood cells and/or populations of the white blood cells removed by elimination of populations and/or subsets thereof with one or more light or electronic parameters.

A fourth application, U.S. Serial No. 07/525,231, filed May 17, 1990 (WO-A-9118086), entitled METHOD AND APPARATUS FOR SCREENING OBSCURED OR PARTIALLY OBSCURED CELLS, discloses an analysis of obscured cells by utilizing microspheres having specific monoclonal antibodies bound thereto to move the sensed characteristics of the obscured cells from one cell population area on a scattergram to another area.

An improved analytical hematology instrument and methods of utilizing the same are disclosed in U.S. Serial No. 025,442 filed March 13, 1987 and continuing U.S. Serial No. 129,954 filed December 4, 1987 (WO-A-8807198), both entitled MULTI-PART DIFFERENTIAL ANALYZING APPARATUS UTILIZING LIGHT SCATTER TECHNIQUES. This so-called VCS hematology instrument utilizes light scattering and electronic sensing techniques to obtain a multi-part differentiation of the leukocyte (L) WBC population. This hematology instrument, however, does not perform differentiation of L subsets, since such subsets are obscured in the L population.

Another related application, U.S. Serial No. 617,075, filed November 23, 1990 (WO-A-9209682), entitled METHOD AND APPARATUS FOR SCREENING MICROSCOPIC CELLS UTILIZING LIGHT SCATTER TECHNIQUES, utilizes light scattering techniques to obtain both L subsets and overlapping subset determinations.

Selectively attaching microscopic particles to each cell of a cell population makes possible the modification of the parameter(s) responsible for the original location of at least one of the populations. The addition of a plurality of microscopic particles to each cell of selected target populations where this addition affects the measured volume and/or opacity results in shifting the location of the dots in the scattergram representing a population.

Antibodies of known specificity are employed in coating microscopic particles. This coating gives the particle the capacity to selectively attach to certain cells which express the antigen the antibody is specific for. These coated or tagged cells are a combination of particles and cell which behave like a new entity. Their parameters of opacity, volume, or both opacity and volume may be considered to represent the sum of the effects of both the cell and the particles on the signals obtained. If the characteristics of the components are different, the new entity will move to a new position on a scattergram in accordance with the net effect. The new location, in contrast with the former position of the cell alone, should allow a classification of such new entity or group of new entities. If the particles attached to the cells are magnetic, then, of course, according to current practice, the new entities can be captured by the use of a magnet. If mixed rapidly, unexpected results including complete capture of a population without adversely affecting the properties of the cells under study occur.

Only three distinct populations of cells can be readily identified and enumerated from a blood sample by utilizing their inherent and unique properties of DC volume and opacity parameters heretofore stated. Additional steps such as improved lysing systems, must be taken to enable the detection and enumeration of more populations. Of course, these additional populations represent subpopulations of the three basic ones referred to as lymphocytes, monocytes and granulocytes. The steps performed in accordance with the above referenced applications demonstrate how subpopulations of these basic three populations are obtained.

Employing such simple aperture sensing techniques in combination with two or more biological particles, one can produce a unique and new position of the dot cluster representing a given population. This selective movement of populations on the dot plot or scattergram is reproducible and can be used to classify a population separate from the basic three populations.

The original and inherent combination of DC volume and opacity sensing techniques can be modified through the attachment of microscopic particles to selected individual cells. The selectivity is given the particles by the nature or specificity of the biological molecules, antibodies among others, employed as the coating on the particle surfaces. A population of cells alone, having no particles on their surface, may occupy a dot plot position no different from other populations or subpopulations, and, henceforth, not be distinguishable from one another. The addition of particles having a selective attraction to a specific population of cells which one seeks to identify, enumerate, and study is possible using this approach. The selective addition of a sufficient mass of selective particles to a distinct population of interest results in the shifting of that population's dot plot location as a result of the new and unique combination of mass, volume and opacity of each cell.

The method and apparatus embodying the invention can be utilized with a variety of immunological reactions, such as immunological reactions involving reactants and formed bodies or cells. The invention also applies to analyses of formed body suspensions such as some bacteria and viruses among others. As utilized herein, cells are defined as animal or plant cells, including cellular bacteria, fungi, which are identifiable separately or in aggregates. Cells are the least structural aggregate of living matter capable of functioning as an independent unit. For example, cells can be human RBC and WBC populations, cancer or other abnormal cells from tissue or from blood samples. Formed bodies are defined as some bacteria and viruses. The cells and formed bodies suitably tagged or labeled, reasonably can be expected to be optically identified by the method and apparatus of the invention in the same manner as the human blood cell examples.

Although the term "reactant" has been utilized in the above applications to define lysing agents and monoclonal antibodies, reactants can include various agents which detect and react with one or more specific molecules which are on the surface of a cell or formed body. Some examples are given below:

| Reactant | Specific Molecule |
|---|---|
| Antibody | Antigen |
| Drug | Drug Receptor |
| Hormone | Hormone Receptor |
| Growth Factor | Growth Factor Receptor |

The reactants couple or bind to the specific molecule(s) on the cells. These reactants do form part of a chemical reaction; however, the reactants are not necessarily chemically altered.

### Disclosure of Invention

A method and apparatus for performing screening of one or more cell groups of interest obscured by a cell population such as one or more subsets of interest of a WBC population. The cell group of interest is enumerated by utilizing microspheres having monoclonal antibodies bound thereto to change the sensed characteristics of specified cells to differentiate the cell group of interest from the obscuring cell population.

A whole blood sample or portion thereof can be screened to provide the desired analysis of a WBC subset of interest. The sample portion is mixed with microspheres having monoclonal antibodies specific to the WBC subset of interest, which microspheres bind to the cells of interest to shift the sensed characteristics of the cells. The sample portion with the WBC subset of interest then is sensed by at least two sensing parameters, one of which is a polarized light sensing parameter and the characteristics of the WBC subset of interest are shifted sufficiently to directly measure the subset of interest. Overlapping cell populations also can be analyzed. Two cell groups of interest can be shifted simultaneously.

A sample portion also can be measured first, then have specified cells deleted therefrom to enable the cell group of interest to be sensed in an area in which it would otherwise have been obscured by the deleted cells. The sample portion is again measured and compared to the first measurement to differentiate the cell group of interest. In a whole blood sample the cell group of interest would be a WBC subset of interest otherwise obscured by a WBC cell population.

### Brief Description of Drawings

The preferred embodiments of this invention will now be described by way of example, with reference to the drawings accompanying this specification in which:
FIG. 1 is a schematic block diagram of one cell screening analyzer embodiment of the present invention;
FIG. 2 is a schematic block diagram of a second cell screening analyzer embodiment of the present invention;
FIGS. 3 is one specific analyzer embodiment of the present invention;
FIG. 4 is one polarized detector arrangement of the present invention;
FIGS. 5A-6D are scattergrams illustrating the techniques of the present invention for obtaining a WBC subset of interest;
FIGS. 7A-7C are scattergrams of results for the WBC subsets of interest utilizing different sensing parameters;
FIGS. 8A-9D are scattergrams illustrating the utilization of two microspheres of the same size in accordance with the present invention;
FIG. 10 is another schematic block diagram of one cell screening analyzer embodiment of the present invention for determining overlapping subsets;
FIG. 11 is a schematic block diagram of a cell screening analyzer embodiment of the present invention for determining obscured cells;
FIG. 12 is a schematic block diagram of another cell screening analyzer embodiment of the present invention for determining obscured cells;
FIG. 13 is a schematic block diagram of a cell screening analyzer embodiment of the present invention for concurrently analyzing two cell groups of interest; and
FIG. 14 is a schematic block diagram of a second cell screening analyzer embodiment of the present invention for concurrently analyzing two cell groups of interest.

### Mode For Carrying Out The Invention

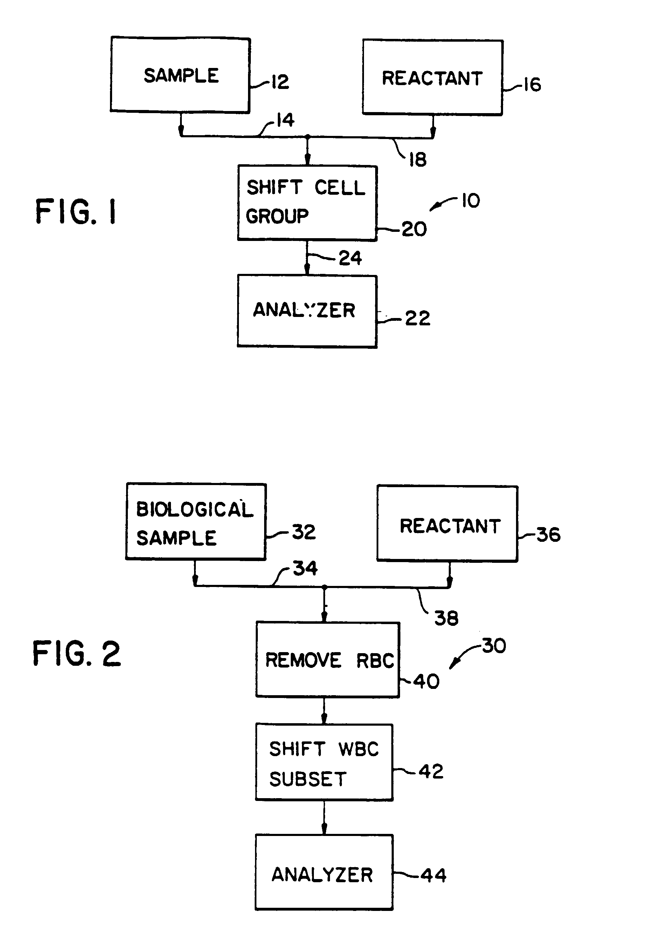

Referring to FIG. 1, a first cell screening analyzer embodiment of the present invention is designated generally by the reference numeral 10. The analyzer 10 includes a sample 12, which contains at least a first set or population of cells (not illustrated). This cell population obscures a cell group of interest, such as a subset or second set of cells, when analyzed as described hereinafter. The sample 12 can include a buffer into which the cells are added.

The sample 12 or a portion thereof is combined via a line 14 with at least one reactant 16 via a line 18. The cells of the cell group of interest have at least one sensed cell characteristic modified or shifted in a shift cell group station 20. The sensed characteristic of the cells of the cell group of interest is modified or shifted sufficiently to remove the cell characteristics from the sensed cell characteristics of the obscuring cell population.

The reactant 16 can be or can include a plurality of microspheres with an antibody bound thereto which is specific to the cell group of interest. The reactant 16 and the sample portion 12 preferably are mixed together to enhance the binding of the microspheres to the cell group of interest. A plurality of the microspheres are bound to each cell of the cell group of interest, generally coating each cell with microspheres, which modifies or shifts the resultant sensed characteristics of each cell of the cell group of interest. The sample portion 12 then is fed into an analyzer 22 via a line 24. The analyzer at least senses and counts the number of cells in the sample portion 12. The analyzer 22 includes at least one and preferably two sensing parameters, at least one of which is a polarized optical parameter, as will be further described hereinafter.

Referring to FIG. 2, a second cell screening analyzer embodiment of the present invention is designated generally by the reference numeral 30. The analyzer 30 includes a biological sample 32 which contains at least a first set of biological cells (not illustrated), such as in or from a whole blood sample. The cells of the biological sample 32 are to be involved in a biological reaction in a quantitative and/or qualitative determination or analysis. The biological sample 32 includes at least one WBC population, which WBC population obscures a WBC subset population of interest. The sample 32 can also include a buffer into which the cells are added.

As utilized herein, WBC subset populations are subsets of a WBC population to which specific monoclonal antibodies can be bound. A nomenclature now has been defined for the monoclonal antibodies by the World Health Organization and the International Immunology Society. The monoclonal antibodies are defined by a cluster designation (CD) nomenclature which defines a particular specificity for a cell or group of cells and the monoclonal antibodies specific for that CD group. For example purposes only, three CD groups have been utilized in the following examples, CD2, CD4 and CD8. The CD nomenclature, specificity and some commercial sources of monoclonal antibodies are illustrated in Table I.

**TABLE I**

| Cluster of Differentiation | Antibody (Commercial Source)^{b} | Specificity |
|---|---|---|
| CD2(gp 50)^{a} | T11 (Coulter) OKT11 (Ortho);Leu5a (BD) | E Rossette Receptor |
| CD4(gp 56) | T4 (Coulter) OKT4ₐ (Ortho);Leu3a (BD) | Helper/inducer T |
| CD8(gp 32-33) | T8 (Coulter) OKT8 (Ortho);Leu2a (BD) | Cytotoxic/ Suppressor T |

| | | |
|---|---|---|
| ^{a} gp - glycoprotein, molecular weight in kilodaltons | | |
| ^{b} Coulter - Coulter Immunology Division of Coulter Corporation (Hialeah, Florida) BD - Becton-Dickinson Immunocytometry Systems Ortho - Ortho Diagnostic Systems (Raritan, New Jersey) | | |

The sample 32 or a portion thereof is combined via a line 34 with at least one reactant 36 via a line 38. The red blood cells (RBC) then are removed from the mixture by a functionally designated RBC removing station 40. The RBC's can be removed from the mixture by the station 40 in a number of ways. The RBC's can be lysed by a lyse in the reactant 36. One such preferential lyse and a quench which can be utilized therewith is disclosed in Serial No. 130,911 (WO-A-8807187), filed December 10, 1987 entitled METHOD AND REAGENT SYSTEM FOR ISOLATION, IDENTIFICATION AND/OR ANALYSIS OF LEUKOCYTES FROM WHOLE BLOOD SAMPLES, which is a CIP of Serial No. 025,303, filed March 13, 1987, of the same title. The reactant 36 can be or can include a plurality of magnetic microspheres with an antibody specific to the RBC's bound to the microspheres (not illustrated). In this example, the particular red blood cell specific antibody utilized is disclosed in U.S. Patent No. 4,752,563, entitled MONOCLONAL ANTIBODY FOR RECOVERY OF LEUKOCYTES IN HUMAN PERIPHERAL BLOOD AND METHOD OF RECOVERY EMPLOYING SAID MONOCLONAL ANTIBODY, which is incorporated herein by reference. The reactant 16 also can include a buffer in addition to or in place of the sample buffer. The reactant 36 further can be a combination of the preferential RBC lyse and the RBC specific microspheres.

Once the RBC's substantially are removed from the sample portion mixture, a portion of the mixture is fed into a subset shift station 42. As in the analyzer 10, the WBC subset population of interest will have at least one sensed cell characteristic modified or shifted in the station 42 to remove the sensed characteristics of the WBC subset population of interest from the sensed cell characteristics of the obscuring WBC population.

Again, the reactant 36 can be or can include a plurality of microspheres with an antibody bound thereto which is specific to the WBC subset population of interest. The reactant 36 and the sample portion 32 preferably are mixed together to enhance the binding of the microspheres to the WBC subset population of interest. A plurality of the microspheres are bound to each cell of the WBC subset population of interest, coating each cell with microspheres, which modifies or shifts the resultant sensed cell characteristic of each cell of the WBC subset population of interest.

The sample portion then is fed into an analyzer 44 which can be identical to the analyzer 22, again at least sensing and counting the number of cells in the sample portion 32, utilizing at least a polarized light parameter.

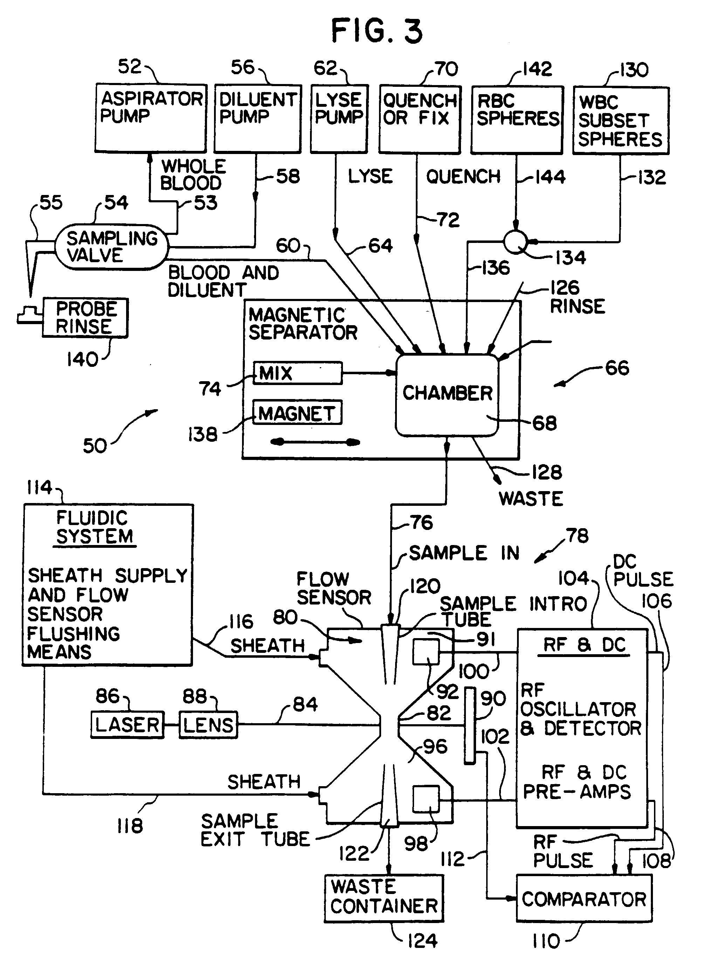

One specific embodiment of an analyzer instrument embodying the present invention and which can accomplish the analyzing methods of the first and second analyzers 10 and 30, is designated generally by the reference numeral 50 in FIG. 3.

In the instrument 50, only one specific enumeration is illustrated, which can be varied in almost endless detail in accordance with the principles of the present invention. Further, the instrument 50 is shown in generally functional detail and the specific embodiments can be structurally implemented in many known ways.

The instrument 50 includes an aspirator pumping mechanism 52 which is utilized to draw the cell sample of interest, for example the sample 12 or 32 into the instrument 50. The aspirator 52 is coupled via a line 53 to a sampling valve 54, which can be coupled to a sample probe 55. A diluent pump 56 can include a diluent or buffer solution and also is coupled to the valve 54 via a line 58. The valve 54 and the pump 52 can aspirate the cell sample 12 or 32 along with the diluent via the pump 56 when appropriate.

The reactant mixture or the cell sample itself, then is fed via a discharge line 60 into a mixing apparatus 66. The mixer 66 includes a mixing chamber 68 into which the sample or reactant is fed. The RBC's then will be lysed by a lyse from a lyse pump 62 fed into the chamber 68 via a line 64. When the reaction is completed a quench or fix then is supplied from a station 70 via a line 72. The reaction can be assisted by mixing the lyse and/or the quench and the sample in the chamber 68 as illustrated functionally at 74.

Specific details of an appropriate mixing apparatus 66, which can be utilized herein are disclosed in Serial No. 025,337, filed March 13, 1987 (WO-A-8806918), and Serial No. 517,309, filed May 1, 1990 (US-A-5238812, published 24.08.93), which is a continuation of Serial No. 025,337 both entitled METHOD AND APPARATUS FOR RAPID MIXING OF SMALL VOLUMES FOR ENHANCING BIOLOGICAL REACTIONS. By utilizing the mixer 66 the reactions are greatly enhanced in speed without significantly damaging the properties of interest of the cells, such as, can occur by raising the reaction temperature. Further, the reactions generally are completed in a significantly reduced time, generally on the order of two minutes or less. This allows a rapid analysis for the automatic high volume analyzer instrument 50.

The quenched sample portion with the RBC's removed by the lyse can be fed via a line 76 directly to a WBC analyzer 78 (i.e., analyzer 22 or 44). The sample portion is fed directly to the analyzer 78 where a reference or non-shifted result is first obtained from the sample for later reference or comparison purposes. The analyzer 78 can be of many physical types in accordance with the counting and sizing techniques described by Wallace H. Coulter in U.S. Patent No. 2,656,508 and embodied in the numerous commercial blood cell counters of the assignee, Coulter Electronics, Inc.

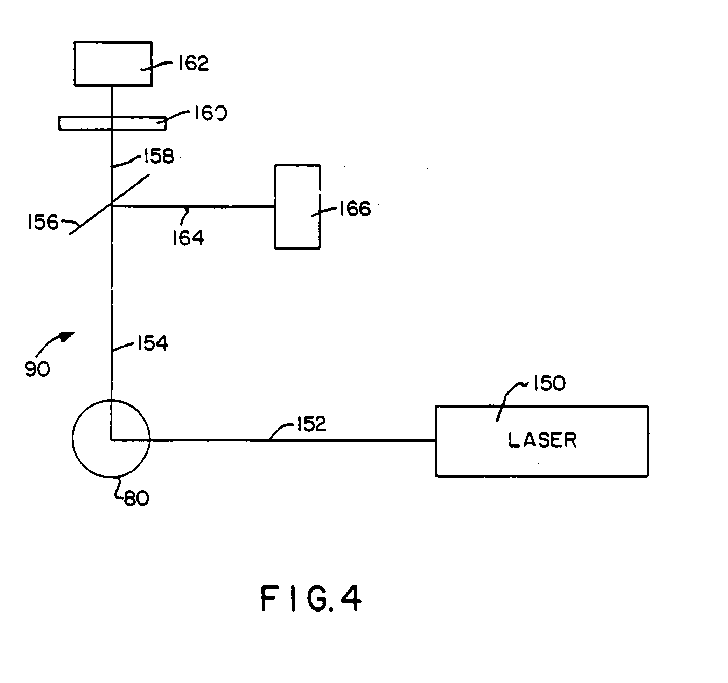

The analyzer 78, in general, includes a flow cell type of sensing chamber 80. The flow cell 80 includes at least polarized optical and preferably electronic sensing devices. The flow cell 80 is formed from any optically transparent material, for example fused silica or quartz. The flow cell 80 includes a narrowed necked-down aperture 82 through which the sample portion flows as a hydrodynamically formed stream in a well known manner. The flow cell 80 includes an optical flat surface for focusing a beam 84 of polarized electromagnetic light energy, preferably from a laser source 86 focused through a lens arrangement 88 into a spot in the aperture 82. The light scattered by the individual cells as they pass through the aperture 82 and hence the beam 84 is detected by a polarized detector arrangement 90, as will be described with respect to FIG. 4.

The flow cell 80 also can include electronic sensing circuitry. The flow cell 80 includes a first portion 91 having a first electrode 92 in contact with the fluid therein.

The flow cell chamber portion 91 and the electrode 92 communicate through the aperture 82 with a second chamber portion 96 having a second electrode 98 therein. The electrodes 92 and 98 are coupled via reactive leads 100 and 102 to an RF/DC source and sensing circuit 104. The circuit 104 couples both a DC, or low frequency current or signal, and a high frequency signal between the electrodes 92 and 98.

The low frequency signal is utilized to sense the amplitude of a signal pulse caused by a cell passing through the aperture 82. The high frequency signal is utilized to obtain the electrical opacity of the same cell passing through the aperture 82.

The measuring of the electrical opacity of cells was described by Wallace H. Coulter and Walter R. Hogg in U.S. Patent No. 3,502,974 and several patents and publications of the assignee, Coulter Electronics, Inc., since that patent. One specific circuit which can be utilized herein is disclosed in U.S. Patent No. 4,791,355, entitled PARTICLE ANALYZER FOR MEASURING THE RESISTANCE AND REACTANCE OF A PARTICLE.

The signals generated by the circuit 104 from the sensed cells are coupled via a DC signal lead 106 and an RF signal lead 108 to a comparator 110. The comparator 110 can hold the signal generated from the sample portions, i.e., for a comparison with the results from the other sample portions to be described. Also, the comparator 110 can include an optical sensing input or inputs via a lead 112 from the optical detector arrangement 90.

The analyzer 78 includes a sheath flow to focus the cells in the flow cell 80, in the well known manner. The sheath flow can be provided by a fluidic system 114, coupled to the flow cell 80 by a pair of lines 116 and 118 in a known manner. The sample reaction mixture can be fed into the flow cell 80 via an introduction tube 120 and can be fed from the flow cell 80 via an exit tube 122 into a waste container 124.

While the first portion of the mixture was being analyzed in the analyzer 78, the mixer 56 can be cleaned or flushed via a rinse line 126 and exhausted through a waste line 128. A second sample portion now can be fed into the chamber 68 to have its sensed cell characteristics modified or shifted. Alternatively, the sample portion to be modified can first be fed into the chamber 68, without a first non-shifted or standard sample portion result first being obtained. The WBC or other cell group of interest now is shifted by adding the WBC microspheres from a station 130 via a line 132, a valve 134 and a chamber line 136 into the chamber 68 to be mixed with the sample portion.

The WBC microspheres are mixed with the second portion by the mixing mechanism 74. For shifting, the WBC microspheres and the reaction mixture with the bound WBC microspheres is fed via the line 76 into the analyzer 78 (i.e., the analyzer 22 or 44), wherein the second portion is analyzed like the first portion and the results then can be compared in the comparator 110. At least one of the WBC subset cell sensed cell characteristics is changed in the second portion, such as the cell opacity by the WBC subset bound microspheres to provide the changed results which then can be analyzed.

If the WBC microspheres are magnetic, for reasons described hereinafter, then the WBC subset population bound thereto can be removed by a magnetic field during and/or after the mixing process by a magnetic field or magnet 138. The field can be provided by electromagnetic means or by the magnet 138 being physically moved with respect to the chamber 68 to capture the magnetically bound WBC subset. The second portion without the bound WBC subset then is fed via the line 76 to the analyzer 86 in the manner previously described to obtain the analysis.

The instrument 66 then is prepared to take the next sample or sample portion for the next analysis. The probe 58 can be cleaned by a probe rinse mechanism 140 and the lines and chamber 68 can be flushed in a conventional manner. Each analysis of the succeeding sample mixture is obtained in a rapid and automatic fashion. The period between the analysis of succeeding sample mixtures can be on the order of minutes or less.

In operating the analyzer instrument 66, the reaction mixture with the RBC lyse/reactant and the sample portion is mixed in the chamber 68 along with non-magnetic WBC microspheres from the station 130, which bind to one of the WBC subsets. The quench 70 is added to the reactive mixture which then is fed via the line 76 to the WBC analyzer 78 for analysis.

Alternatively to the utilization of the lyse, the sample 12 or 32 can be fed to the mixer 66 via the valve 56 without lyse. In this case, the RBC's can be removed magnetically by utilizing the microspheres with the RBC specific antibody bound thereto from an RBC microsphere station 142 and fed to the valve 134 via a line 142 and hence to the chamber 68 via the line 136. Where lyse is not utilized, the bound RBC's are magnetically removed by the magnet 140 after mixing in a manner substantially identical to the magnetically bound WBC's described above.

Further, in a second case to promote the speed of the reaction, a reaction mixture of the sample with both the RBC lyse and with the RBC magnetic beads can be utilized. The reaction mixture is mixed, the lyse is quenched and the bound RBC's are magnetically removed and then the WBC's are analyzed as previously described.

Although the technique of the present invention has been described utilizing a sample in its natural state, such as a whole blood sample fed directly into the instrument 66, the sample also can be off line prepared or partially pre-prepared where desired. The sample could have the RBC's previously deleted. A system can be utilized in which the microspheres could be added off line or in a preparation mode and the sample portion with the sensed cell characteristics already shifted then could be introduced into the system for the rest of the analysis. Some specific examples are described hereinafter.

Leukocyte or WBC population differentiation utilizing multi-dimensional light scattering is disclosed in a CELL-DYN 3000 instrument sold by Sequoia-Turner of Mountainview, California. The instrument utilizes a polarized light source and measures forward, ten degree and ninety degree (orthogonal) polarized and depolarized light scatter. The instrument also utilizes a reagent formulation to make the RBC's "transparent" to the laser light. However, even with these multiple parameters, the instrument does not provide any WBC subset population differentiation.

Referring to FIG. 4, one example of the polarized optical detector arrangement 90 is illustrated. The arrangement 90 includes a polarized laser source 150 which directs a polarized light beam 152 to the flow cell 80. The light beam 152 interacts with the cell stream (not illustrated) and forms a reflected beam 154 containing information relative to the cells. The reflected beam 154 is directed to a beam splitter 156.

A first portion 158 of the beam passes through the beam splitter 156, through a polarized filter 160 and is detected by a photomultiplier tube (PMT) 162 as a 90 degree polarized light scatter signal or output. A second portion 164 of the beam is reflected from the beam splitter 156 and is directed to and detected by a second PMT 166 as a non-polarized light scatter signal. Other light sensing parameters also can be utilized such as disclosed in the above-referenced VCS and Case 35368 instruments.

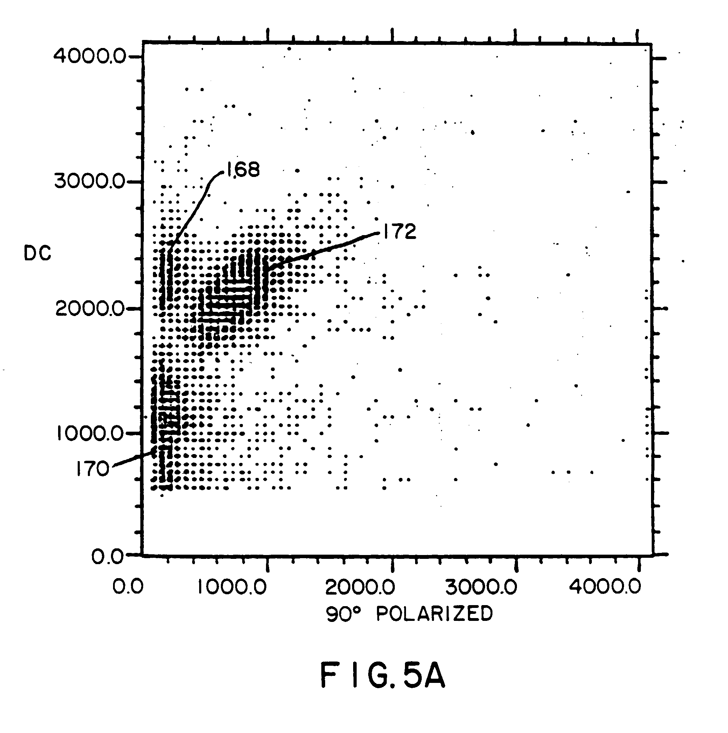

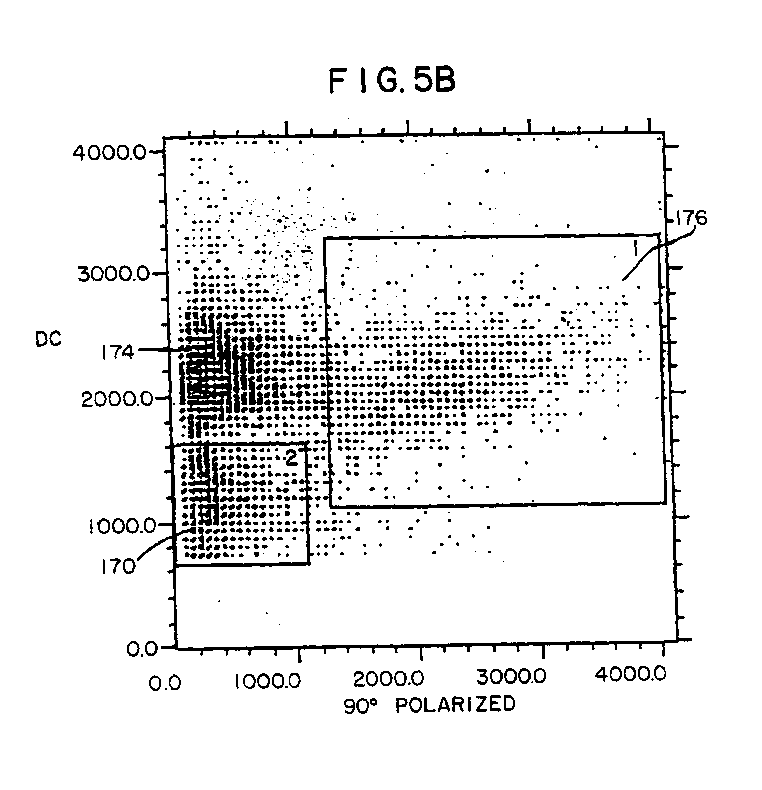

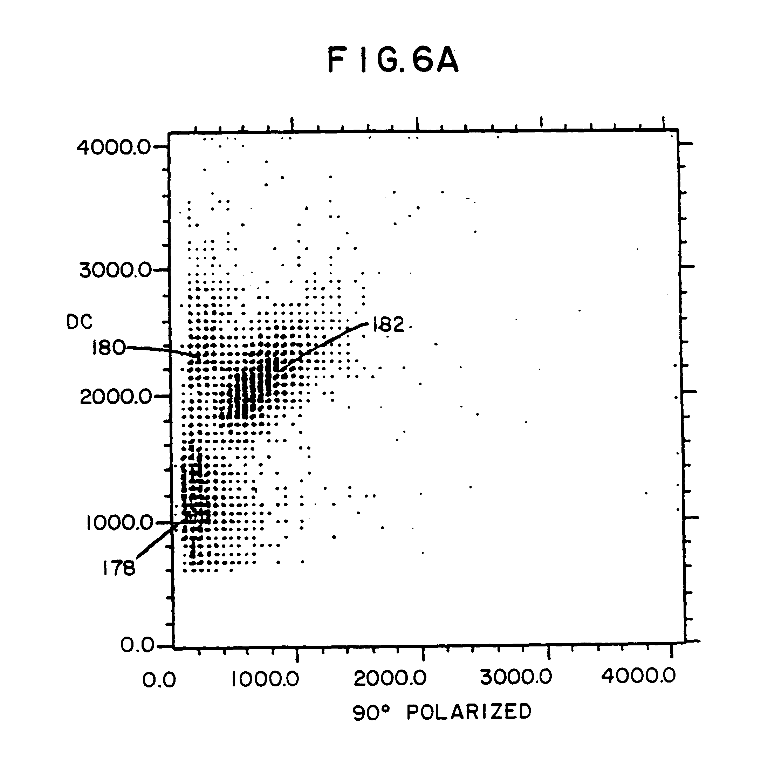

Referring to FIGS. 5A-6B, a scattergram provides, on a DC/polarized light scatter plot as shown in FIG. 5, three WBC population groupings, 168 monocytes (M), 170 L's and 172 neutrophils (N) and eosinophils (E) combined. In this scattergram, no microspheres are present.

When microspheres are added, the light scattered by the microspheres blends the sensed characteristics such that only two WBC population groupings are separately enumerated as shown in FIG. 5B, 170 L's and 174 M's, N's, E's combined. This blending of the cell groupings can be minimized by utilizing a minimum number of microspheres. Further, very small microspheres, on the order of less than 0.6 micron diameter do not cause any significant blending utilizing a light source having a wavelength of 488 nm.

Utilizing FIG. 5A as the standard or control, then non-magnetic microspheres with a monoclonal antibody bound thereto specific to an L subset, such as CD4, are mixed in the same sample or in a second portion of the sample resulting in a shift forming a direct subset grouping (176) as shown in FIG. 5B.

This procedure can be utilized off-line or on-line. Steps 1-3 of the procedure could be accomplished off-line. This would eliminate the equipment necessary to add the microspheres and mix them with the sample. A fully automatic instrument 50 could accomplish all the steps automatically on-line. The procedure, in general, is as follows:
1. Provide 150 microliters of whole blood (or more).
2. Add 15 microliters of non-magnetic 2 micron microspheres having one L subset specific monoclonal antibody bound thereto (T4, T8 or Tll each in a separate mixing vessel) or add 10 microliters of MSIG control microspheres to form control/background standard.
3. Mix 2 minutes (can stand for a period of time afterwards, at least up to an hour).
4. Run (automatic) in system.
5. Lyse and quench on-line in system.

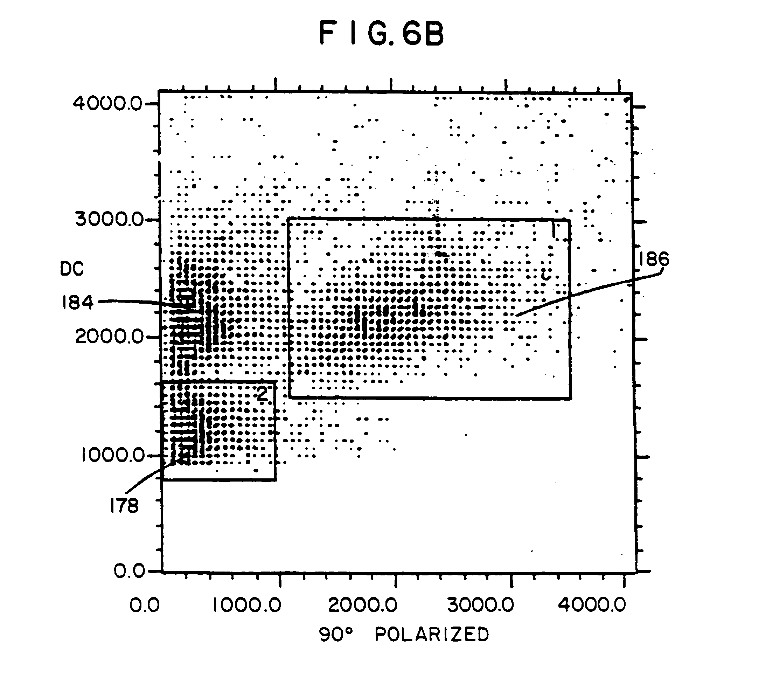

Referring to FIG. 6A, a control or non-shifted scattergram result again is illustrated. The L's are found in a grouping 178, while the M's are found in a grouping 180 and the N's and E's are found in a grouping 182. As stated above, the control need not be provided, since as can be seen in FIGS. 5B and 6B, the WBC subset of interest can be located directly and therefor there is no need to compare back to a reference result. The MSIG control microspheres only are utilized to ensure that the scattergram pattern is free of interference. MSIG is a mouse monoclonal immunoglobin having no specificity for any WBC or WBC subset population.

A first WBC subset of interest, the CD4 cells are shifted by binding T4 microspheres thereto as illustrated in FIG. 6B. The remaining L's without the shifted subset are shown in a grouping 178, while the M's, N's and E's now are found in a group 184. The shifted sensed cell characteristic of the CD4 cells, form a new grouping 186, which can be compared to the results of FIG. 6A or can be analyzed directly as a percentage of CD4 cells to the total L's. In this example, the percentage contribution of the CD4 cells was found to be 53.2 percent. The same sample was analyzed in a conventional EPICS (registered trademark) flow cytometry instrument, for comparison purposes, which resulted in a percentage contribution of 53.3 percent.

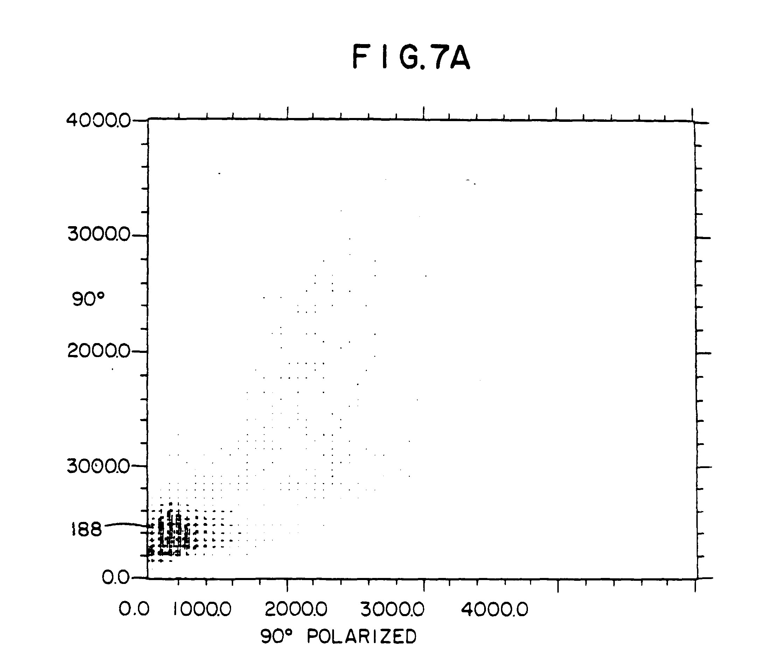

FIGS. 7A-7C illustrate the CD4 shift, utilizing different sensing parameters to form the scattergrams. These results are not optimized, but are illustrated to show the principle of utilizing other sensing parameters such as MAS (medium angle scatter - not illustrated in FIGS. 3 and 4). FIG. 7A illustrates a control cell grouping 188 for all the WBC populations. Ninety degree light scatter is plotted against 90 degree polarized light scatter in this scattergram.

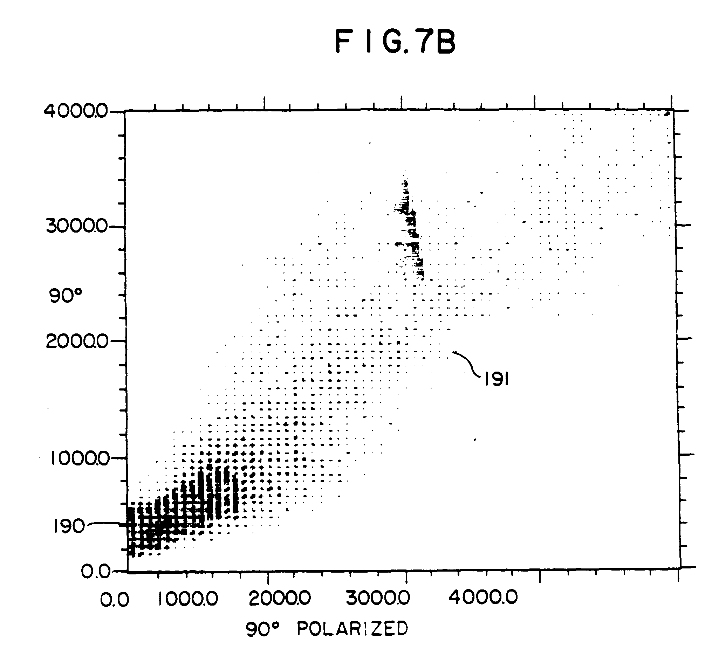

FIG. 7B illustrates a single cell grouping 190 for all the WBC populations and a scattered CD4 grouping 191 for the shifted CD4 cells from the same sample mixture. In this scattergram 90 degree depolarized light scatter again is plotted against 90 degree polarized light scatter.

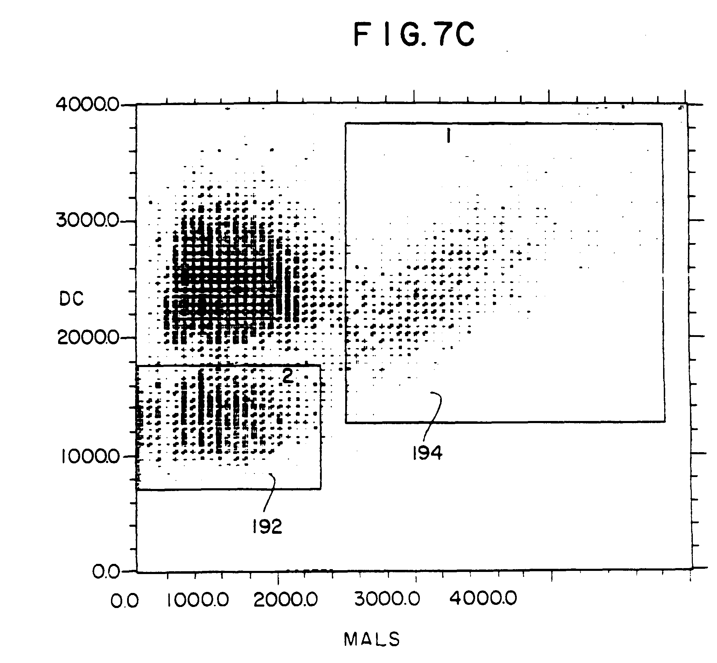

FIG. 7C illustrates the same data as that in FIG. 7B, with a scattergram formed by plotting DC against MALS. The L's are shown in a grouping 192, while the shifted CD4 cells are shown in a grouping 194.

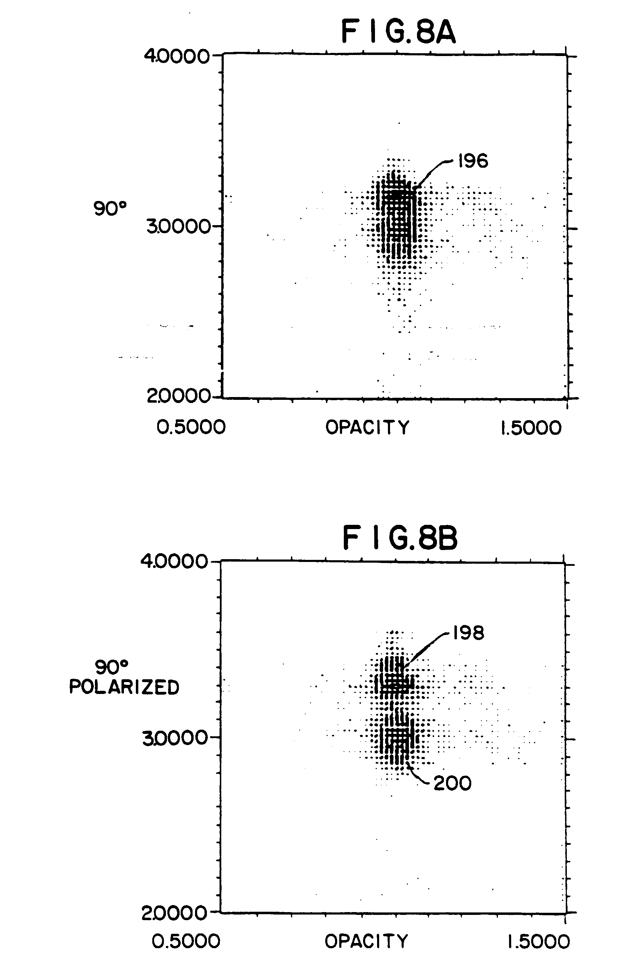

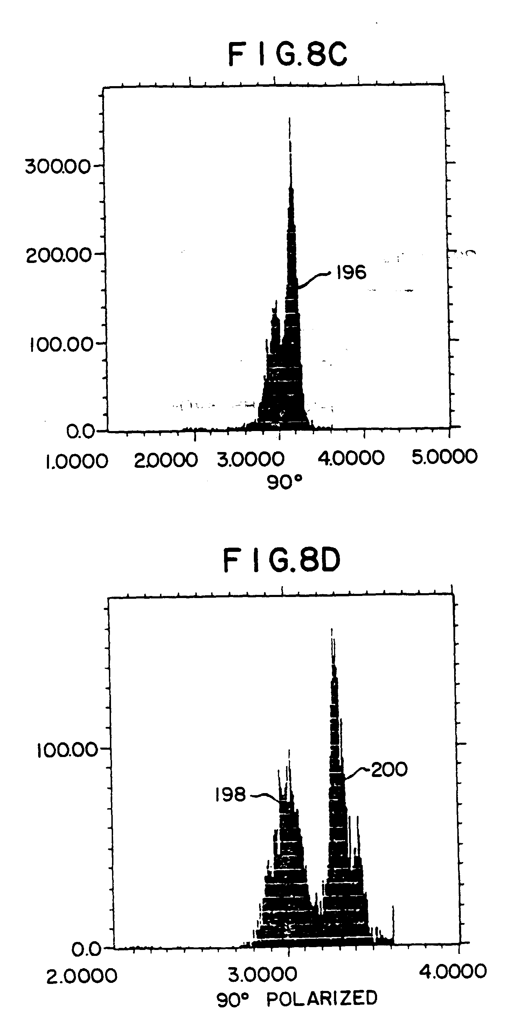

FIGS. 8A-D illustrate the potential feasibility of concurrently utilizing two microspheres of the same diameter to screen two different cell groups of interest, such as two WBC subset populations of interest. Utilizing two different types of microspheres, one 5.74 microns and one 5.11 microns and log non-polarized light scatter versus opacity, as illustrated in FIG. 8A, only a single microsphere grouping 196 is obtained. However, utilizing the polarized sensing parameter log 90 degree polarized light scatter, as illustrated in FIG. 8B, two separate groupings 198 and 200 are obtained. Utilizing these parameters, two separate WBC subset populations potentially could be shifted and results obtained in one sample portion. The results illustrated do not include any cells, but only the microspheres themselves. Further, as illustrated in FIGS. 8C and 8D, utilizing only the single polarized parameter log 90 degree polarized light scatter, results in a one-dimensional separation between the two microsphere groupings (FIG. 8D). The regular non-polarized light scatter shows no differentiation between groupings (FIG. 8C).

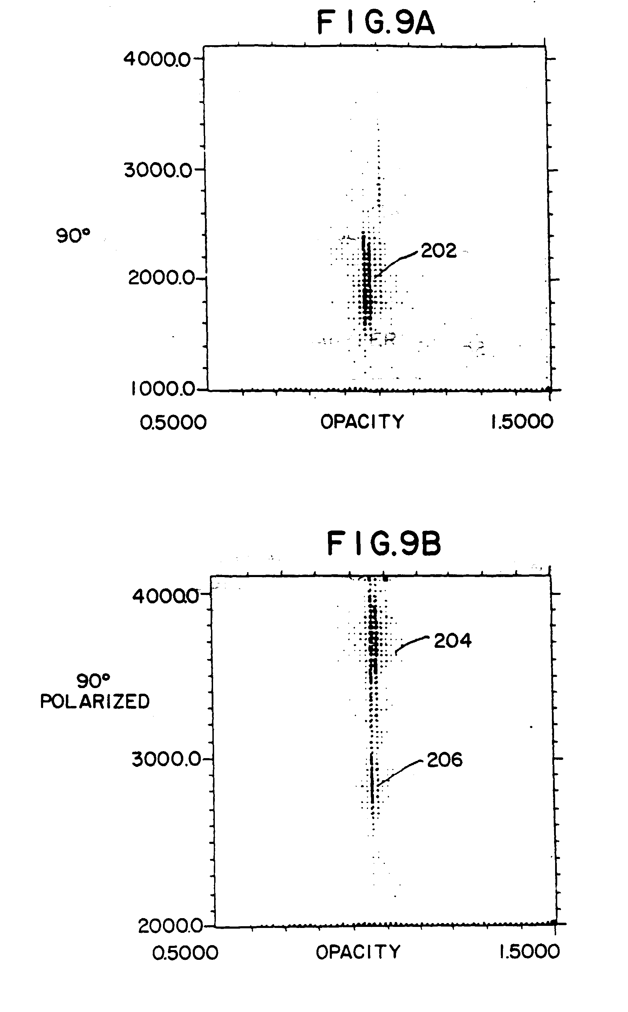

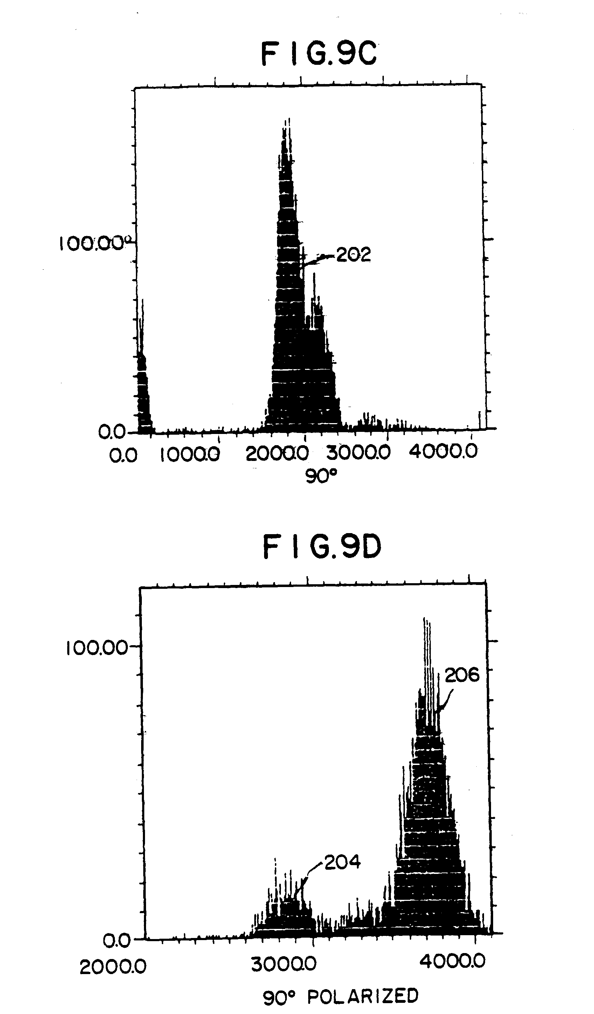

Two other types of microspheres, one 10 microns and one 10.35 microns, also illustrate the potential separation utilizing polarized light, as illustrated in FIGS. 9A-9D. FIG. 9A illustrates only a single grouping 202 with 90 degree scatter versus opacity, while FIG. 9B illustrates two separate groupings 204 and 206 utilizing polarized light scatter. Again, these can also be obtained with a single polarized sensing parameter as illustrated in FIG. 9D. The non-polarized data in FIG. 9C does not show two groupings.

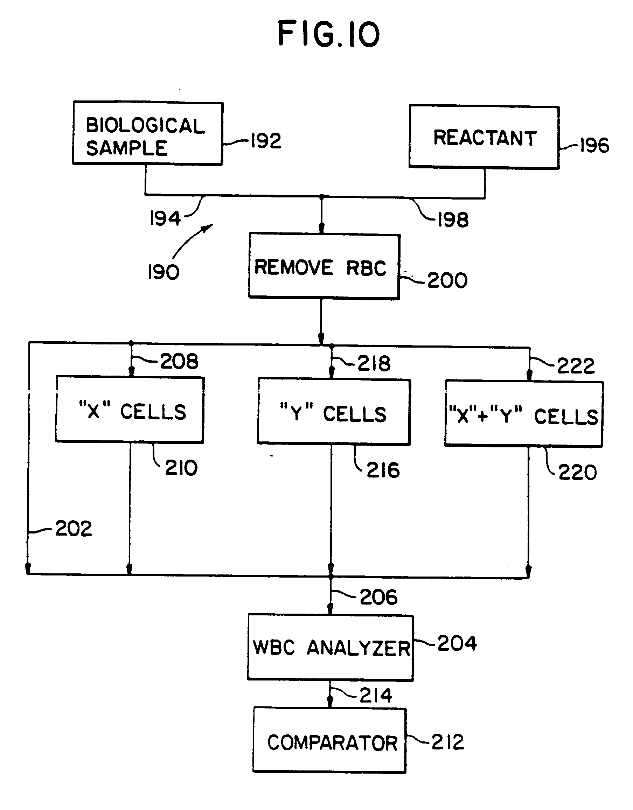

Referring to FIG. 10, a third cell screening analyzer embodiment of the present invention is designated generally by the reference numeral 190. The analyzer 190 is utilized to determine the percentage overlapping of the cell groups of interest and will be described with respect to WBC population subsets of interest from a whole blood sample or portion thereof.

The analyzer 190 includes a biological sample 192, which again contains at least a first WBC population, including at least two WBC population subsets of interest. The biological sample 192 or a portion thereof is combined via a line 194 with at least one reactant 196 via a line 198. The RBC's then are removed from the sample portion by a functionally designated RBC removing station 200. The RBC's are removed by one of the techniques previously described.

Once the RBC's are removed, portions of the mixture can be fed to different lines. A first line 202 can be utilized to deliver a first sample portion directly to an analyzer 204 via a line 206. As with the above described analyzers, 22 and 44, the characteristics of the cells are sensed by at least two sensing parameters, one of which is a light sensing parameter.

A second line 208 delivers a second sample portion to an "X" cell shifting station 210. As before, the "X" cells are a first WBC population subset of interest which will have at least one sensed cell characteristic modified or shifted in the station 210 to remove the sensed characteristics of the WBC population subset of interest from the sensed cell characteristics of the obscuring WBC population. The sensed cell characteristics are shifted by binding a plurality of non-magnetic microspheres with an antibody specific to the WBC population subset of interest to the cells, as before described. The sample portion then is fed to the analyzer 204. Each of the sets of data obtained by the analyzer 204 can be stored for later comparison in a comparator 212 via a line 214.

In a like manner, a third sample portion is fed to a "Y" cell shifting station 216 via a line 218. The sensed cell characteristics of the "Y" cells are shifted or modified in the station 216 and the sample portion then is fed to the analyzer 204 and the comparator 212. This enables the obtaining of the percentage of the "X" cells and the "Y" cells either directly, or by comparison with the non-shifted results from the line 202. This, however, does not enable the obtaining of how many, if any, of the "X" and "Y" cells overlap.

To determine the overlapping percentage, a fourth sample portion is fed to an "X" and "Y" shifting station 220 via a line 222. Overlapping is utilized herein to signify that certain cells, populations of cells, subpopulations of cells or formed bodies include at least two receptors or antigens of interest. In this sample portion, both microspheres having an antibody specific to the "X" cells or first WBC population subset of interest and microspheres having an antibody specific to the "Y" cells or the second WBC population subset of interest are combined together. The sample portion then is fed to the analyzer 204 and the data to the comparator 212.

The overlapping percentage is found by adding the separate "X" and "Y" percentage results from lines 208 and 218 together. From this total, the combined "X" and "Y" percentage result from line 222 is subtracted. If the two total percentages substantially are equal, the "X" and "Y" cells do not significantly overlap. If there is a difference, the difference is the percentage overlap of the cells to which both the "X" and "Y" specific microspheres will bind. Although the analyzer 290 has been described having a plurality of lines, the analyzer 290 also can be a sequential analyzer utilizing a single line such as described with respect to the analyzer 50. The plurality of lines also can be provided in accordance with the analyzer 50, utilizing separate mixers 66 for each line 208, 218 and 222 arranged in parallel. Specific results of this procedure are provided in FIGS. 9A-9F.

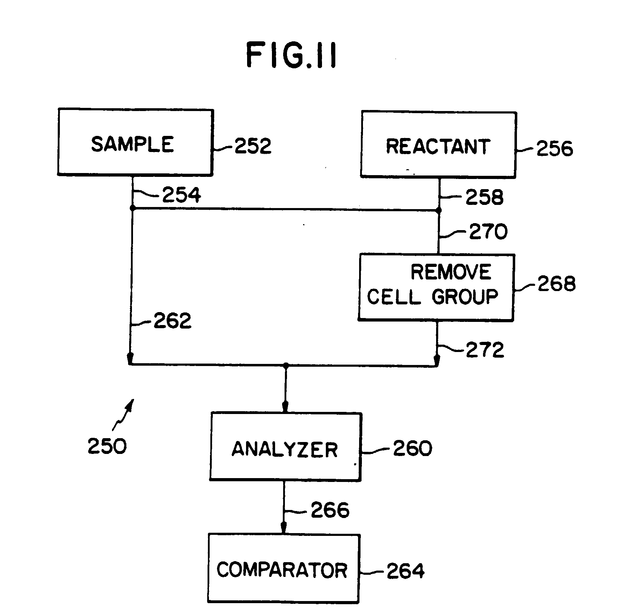

Referring now to FIG. 11, another obscured cell screening analyzer embodiment of the present invention is designated generally by the reference numeral 250. The analyzer 250 includes a sample 252, which contains at least a first set or population of cells (not illustrated). This cell population obscures a cell group of interest, such as a subset or second set of cells, when analyzed as described hereinafter. The sample 252 can include a buffer into which the cells are added.

The sample 252 or a portion thereof is combined via a line 254 with at least one reactant 256 via a line 258. A first portion of the mixture is fed directly to an analyzer 260 via a line 262. The analyzer 260 can be the same as the analyzer 22 and at least senses and counts the number of cells in the sample portion. The results are fed to a comparator 264 via a line 266.

A second sample portion is fed to a cell removing station 268 via a line 270. The cells are removed by shifting or depletion as above described. For shifting, non-magnetic microspheres are bound to the obscuring cell population modifying the sensed cell characteristics sufficiently to remove them from the sensed cell characteristics of the obscured cell population of interest. For depletion, magnetic microspheres are bound to the obscuring cell population which then magnetically are removed from the sample portion.

The second sample portion then is fed to the analyzer 260 via a line 272 and the resulting data is fed to the comparator 264. The data from the two sample portions then is compared to determine the percentage contribution of the obscured cell group of interest.

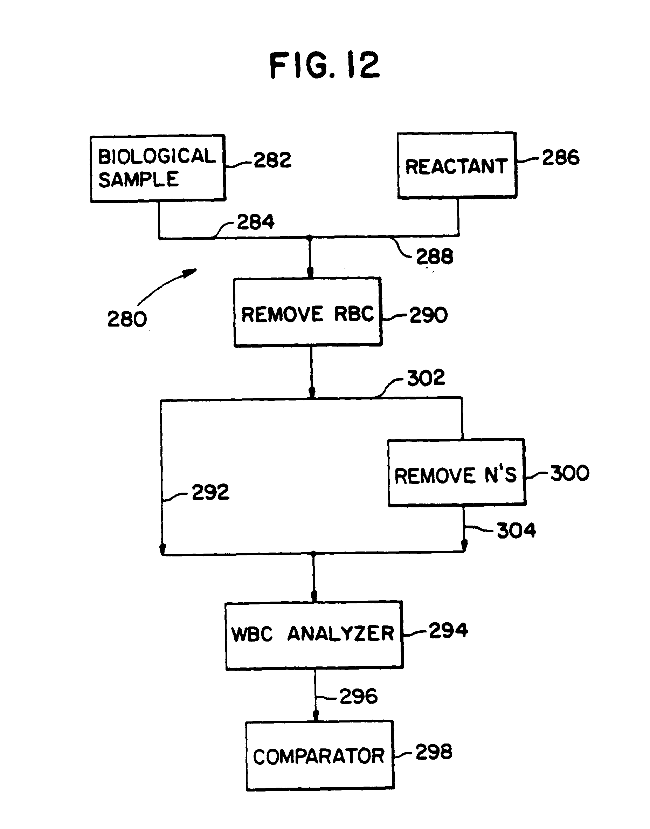

Referring to FIG. 12, another cell screening analyzer embodiment of the present invention is designated generally by the reference numeral 280. The analyzer 280 is similar to the analyzer 250, but includes a biological sample 282 which contains at least a first set of viable biological cells (not illustrated), such as in or from a whole blood sample. The cells of the biological sample 282 are to be involved in a biological reaction in a quantitative and/or qualitative determination or analysis. The biological sample 282 includes at least one WBC population, which WBC population obscures a WBC population of interest. The sample 282 can also include a buffer into which the cells are added.

The sample 282 or portion thereof is combined via a line 284 with a reactant 286 via a line 288. The RBC's are removed from the sample portion 282 in a RBC removal station 290. The RBC's are removed in one of a number of ways previously described. A portion of the RBC removed sample is fed via a line 292 to an analyzer 294, which again at least senses and counts the WBC population. The data from the analyzer 294 is fed via a line 296 to a comparator 298, as in the analyzer 250.

A second sample portion is fed to a neutrophil (N) functionally designated removal station 300 via a line 302. The N's can be removed from the mixture by shifting or changing one parameter, such as opacity, or by magnetic removal, both as described above. In this example, the particular N specific antibody utilized is disclosed in U.S. Patent No. 4,931,395, MONOCLONAL ANTIBODY SPECIFIC TO NEUTROPHILS.

The mixture with the N's removed or shifted then is fed to the WBC analyzer 294 via a line 304. The results of the analyzer 294 are fed to the comparator 298. The results of the second sample portion then are compared to the first sample portion to determine if any cells remain in the N area which previously were obscured by the N's.

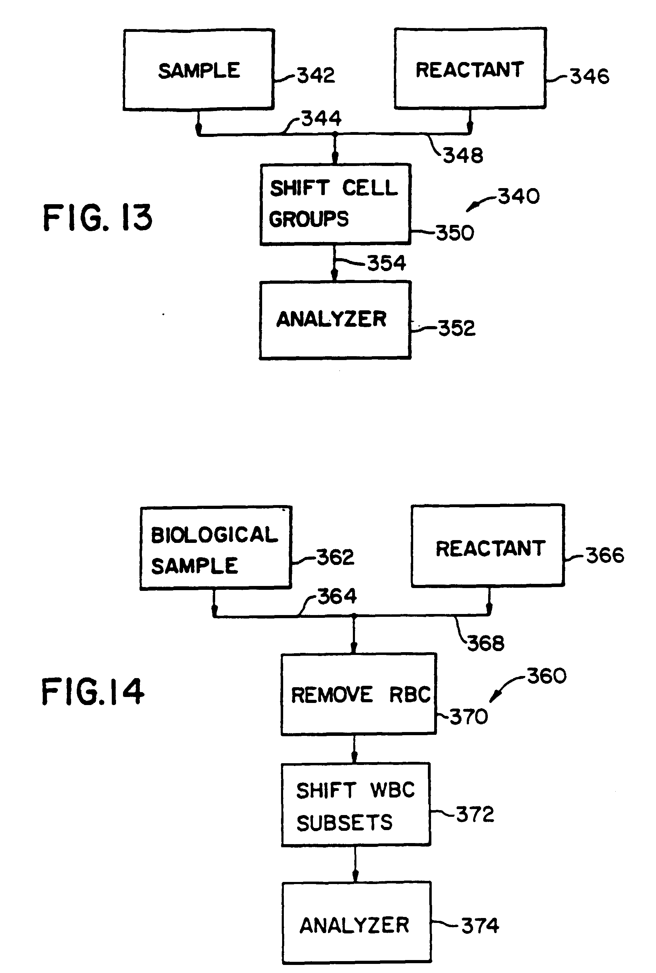

Referring to FIG. 13, a concurrent cell group screening analyzer embodiment of the present invention is designated generally by the reference numeral 340. The analyzer 340 includes a sample 342, which contains at least a first set or population of cells (not illustrated). This cell population obscures at least two cell groups of interest, such as subsets or other sets of cells, when analyzed as described hereinafter. The sample 342 can include a buffer into which the cells are added.

The sample 342 or a portion thereof is combined via a line 344 with at least one reactant 346 via a line 348. Each of the cell groups of interest have at least one sensed cell characteristic modified or shifted in a shift cell groups station 350. Each of the groups of sensed cell characteristics is modified or shifted sufficiently to remove the sensed cell characteristics from the sensed cell characteristics of the obscuring cell population and from one another.

The reactant 346 can be or can include sets of a plurality of different non-magnetic microspheres, each with an antibody bound thereto which is specific to one of the cell groups of interest. The reactant 346 and the sample portion 342 preferably are mixed together to enhance the binding of the microspheres to the cell groups of interest. A plurality of the microspheres are bound to each cell in each cell group of interest, generally coating each cell with microspheres, which modifies or shifts the resultant sensed characteristics of each cell of the cell groups of interest.

The sample portion or mixture 342 then is fed into an analyzer 352 via a line 354. The analyzer at least senses and counts the number of cells in the sample portion 342. The analyzer 352 includes at least two sensing parameters, at least one of which is an optical parameter, such as previously described. The analyzer 340 can be essentially the same as the analyzer 10 and 50, with the addition of at least two different sets of microspheres.

Referring to FIG. 14, a second concurrent cell screening analyzer embodiment of the present invention is designated generally by the reference numeral 360. The analyzer 360 includes a biological sample 362 which contains at least a first set of viable biological cells (not illustrated), such as in or from a whole blood sample. The cells of the biological sample 362 are to be involved in a biological reaction in a quantitative and/or qualitative determination or analysis. The biological sample 362 includes at least one WBC population, which WBC population obscures at least two WBC subset populations of interest. The sample 362 can also include a buffer into which the cells are added.

The sample 362 or a portion thereof is combined via a line 364 with at least one reactant 366 via a line 368. The RBC's then are removed from the mixture by a functionally designated RBC removing station 370. The RBC's can be removed from the mixture by the station 370 in a number of ways, such as the lyse, magnetic microspheres or combination thereof, as previously described.

Once the RBC's substantially are removed from the sample portion mixture 362, a portion of the mixture is fed into a subset shift station 372. As in the analyzer 10, each of the WBC subset populations of interest will have at least one sensed cell characteristic modified or shifted in the station 42 to remove the sensed cell characteristics of the WBC subset populations of interest from the sensed cell characteristics of the obscuring WBC population and from one another.

Again, the reactant 366 can be or can include two sets of different microspheres, each including a plurality of non-magnetic microspheres with an antibody bound thereto which is specific to one of the WBC subset populations of interest. The reactant 366 and the sample portion 362 preferably are mixed together to enhance the binding of the microspheres to the WBC subset populations of interest. As before, a plurality of the microspheres from a set are bound to each cell of each WBC subset population of interest, coating each cell with microspheres, which modifies or shifts the resultant sensed cell characteristic of each cell of the WBC subset population of interest.

The sample portion 362 then is fed into an analyzer 374 which can be identical to the analyzer 22, and again at least sensing and counting the number of cells in the sample portion 32. The analyzer instrument 50 can accomplish the analyzing method of the analyzer 360.

The analyzers also preferably can compute the absolute number of WBC's and hence also the absolute number of the WBC subset populations. This is especially useful for treatment of persons having AIDS since the specific CD4 population number is utilized in deciding how to perform drug treatment of AIDS patients. The absolute number is found by counting the number of WBC in a specific volume in a conventional manner as is performed in many commercial cell counting instruments of the assignee, Coulter Electronics, Inc.

The magnetic microspheres utilized can be of any suitable type and for example, are polystyrene magnetic microspheres of 0.7 micron diameter, with a weight to volume of 10% solids, sold by Bangs Laboratories of Carmel, Indiana. The non-magnetic microspheres again can be of any suitable type and for example, are surfactant free sulfated polystyrene latex microspheres of 2.17 micron diameter with a weight to volume of 8% solids, sold as IDC microspheres by Interfacial Dynamics of Portland, Oregon. Although these specific microspheres are utilized for example purposes, other types and sizes of microspheres from other conventional sources also can be utilized.

In general, for shifting, it is preferable to utilize microspheres of a diameter substantially less than the diameter of the cells, since a plurality of the microspheres should bind to each cell. Typically, microspheres of diameters from 0.65 to 3.0 microns are utilized to ensure that the instrument does not sense and count the free microspheres themselves as an indicated cell. Further, very large microspheres could clog or block the sensing apertures, since a plurality of cells could be bound thereto, resulting in a large microsphere and cell mass. Typically, non-magnetic microspheres are less expensive and hence are utilized for shifting purposes, although magnetic microspheres also can be utilized to cause the sensed cell shift. The range of microsphere sizes utilized also is dependent upon the wavelength of the light utilized.

To eliminate cells magnetically, only magnetic microspheres can be utilized. In this application, the microsphere size is less relevant, since the cells are to be eliminated from the sample before sensing. The microspheres should be of sufficient means to quickly and easily be removed in the magnetic field. In this case, 0.65 to 4.5 micron diameter microspheres work well. Further, since the cells only are to be eliminated, 10 or greater micron diameter microspheres could be utilized. A plurality of cells could be bound to each microsphere, but since no counting will take place, this does not interfere with the operation of the instrument.

## Claims

1. A method of obtaining an analysis of cells of interest in a sample having an obscuring cell population therein, comprising:
shifting the sensed characteristics of the cells of interest with respect to the sensed characteristics of the cells of the obscuring cell population by mixing with a first sample portion microspheres having, bonded thereto, a reactant specific to a specific molecule on the cells of interest, the microspheres being substantially smaller than said cells;
passing the cells substantially one at a time through a sensing zone including a polarised light beam, and sensing and counting the individual cells of the sample portion with a light scatter non-polarised sensing parameter; and
utilising the sensed parameter to characterise the cells and to obtain the contribution of the cells of interest.

2. A method as claimed in claim 1, which additionally comprises sensing and counting the first sample portion, prior to the shifting step, and comparing the respective counts, to obtain the percentage contribution.

3. A method as claimed in claim 1 or claim 2, which additionally comprises shifting the sensed characteristics of a second cell group of interest in the first or a second sample portion by mixing therewith microspheres having, bonded thereto, a reactant specific to a specific molecule on the second cell group of interest.

4. A method of obtaining an analysis of cells of interest in a sample having an obscuring cell population therein, comprising:
passing the cells of a first sample portion substantially one at a time through a sensing zone including a polarised light beam, and sensing and counting the individual cells of the first portion with a light scatter non-polarised sensing parameter;
removing or shifting the cells of the obscuring cell population from a second sample portion by mixing therewith microspheres having, bonded thereto, a reactant which is specific to a specific molecule on the cells of the obscuring cell population;
passing the cells of the second sample portion substantially one at a time through a sensing zone including a polarised light beam, and sensing and counting the individual cells of the second sample portion with a light scatter non-polarised sensing parameter; and
comparing the two counts, to obtain the contribution of the obscured cells of interest.

5. A method as claimed in claim 4, which comprises shifting a sensed characteristic of the cells in the second sample portion, the microspheres being substantially smaller than the cells.

6. A method as claimed in any preceding claim, wherein the sample is of whole blood, the obscuring cell population is a WBC population, and the or each group of cells of interest is a subset population of the WBC population.

7. A method as claimed in claims 3 and 6 which comprises shifting the sensed characteristics of each of the WBC subset populations by mixing both microspheres with a third sample portion, and comparing the results of the first, second and third sample portions, to determine the percentage overlapping of the first and second WBC population subsets.

8. A method as claimed in claim 6 or claim 7, wherein the or each subset is selected from CD2, CD4 and CD8 WBC population subsets.

9. A method of obtaining an analysis of a WBC population in a whole blood sample, comprising:
passing the cells of the WBC population substantially one at a time through a sensing zone including a polarised light beam, and sensing and counting the individual cells with a light scatter non-polarised sensing parameter;
mixing with the sample microspheres having, bonded thereto, a reactant specific to a specific molecule on the cells of a WBC population subset, and removing microspheres with cells bound thereto from the sample;
passing the cells of the remaining WBC population substantially one at a time through a sensing zone including a polarised light beam, and sensing and counting the individual cells with a light scatter non-polarised sensing parameter; and
comparing the two counts, to obtain the remaining and/or the removed WBC population.

10. A method of obtaining an analysis of a WBC population in a whole blood sample, comprising:
passing the cells of the WBC population substantially one at a time through a sensing zone including a polarised light beam, and sensing and counting the individual cells with a light scatter non-polarised sensing parameter;
shifting the sensed characteristics of the cells of a WBC population subset by mixing with the sample microspheres having, bonded thereto, a reactant specific to a specific molecule on the cells of the subset, the microspheres being substantially smaller than the cells;
passing the cells substantially one at a time through a sensing zone including the individual cells with a light scatter non-polarised sensing parameter; and
comparing the two counts, to obtain the remaining and/or shifted WBC populations.

11. A method as claimed in any of claims 6 to 10, wherein the whole blood sample includes a red blood cell population, and which additionally comprises removing the red blood cell population from the sample without significantly adversely affecting relevant qualities and/or quantities of the WBC population.

12. A method as claimed in any preceding claim, wherein the or each reactant is a monoclonal antibody and the or each specific molecule is an antigen.

13. A method as claimed in claim 12, wherein the monoclonal antibodies are neutrophil-specific and the cells of interest comprise an immature WBC population.

14. A method as claimed in any preceding claim, wherein the cells are sensed additionally with one electronic sensing parameter, or with at least two different electronic sensing parameters, to give at least a two-dimensional characterisation of the cells.

## Patentansprüche

1. Verfahren zum Erhalt einer Analyse von interessierenden Zellen in einer Probe mit einer darin vorhandenen abdeckenden Zellenpopulation, wobei folgendes vorgesehen ist:
Verschieben der abgefühlten Charakteristika der interessierenden Zellen bezüglich der abgefühlten Charakteristika der Zellen der abdeckenden Zellenpopulation durch Mischen mit einem ersten Probenteil Mikrosphären oder Mikrokügelchen mit einem damit verbundenen Reaktionsmittel, welches für ein spezielles Molekül an den interessierenden Zellen spezifisch ist, wobei die Mikrokügelchen wesentlich kleiner sind als die erwähnten Zellen;
Hindurchgehen der Zellen, im wesentlichen eine zur jeder Zeit, durch eine Abfühlzone mit einem polarisierten Lichtstrahl, und Abfühlen und Zählen der einzelnen Zellen des Probenteils mit einem lichtstreuenden nicht polarisierten Abfühlparameter; und
Verwenden der abgefühlten Parameter zur Charakterisierung der Zellen und zum Erhalt des Beitrags der interessierenden Zellen.

2. Verfahren nach Anspruch 1, wobei zusätzlich vor dem Verschiebeschritt das Abfühlen und Zählen des ersten Probeteils vorgesehen ist und Vergleichen der entsprechenden Zählerständer zum Erhalt des prozentualen Beitrags.

3. Verfahren nach Anspruch 1 oder 2, wobei zusätzlich die abgefühlten Charakteristika einer zweiten interessierenden Zellengruppe in dem ersten oder einem zweiten Probenteil verschoben werden, und zwar durch Mischen von Mikrosphären oder Mikrokügelchen damit die damit verbunden ein Reaktionsmittel aufweisen, welches für ein spezielles Molekül auf der zweiten interessierenden Zellengruppe spezifisch ist.

4. Verfahren zum Erhalt einer Analyse von interessierenden Zellen in einer Probe mit einer darin befindlichen abdeckenden Zellenpopulation, wobei folgendes vorgesehen ist:
Hindurchlaufen der Zellen eines ersten Probenteils, und zwar im wesentlichen eine zu jedem Zeitpunkt, durch eine einen polarisierten Lichtstrahl aufweisende Abfühlzone, und Abfühlen und Zählen der einzelnen Zellen des ersten Teils mit einem lichtstreuenden nicht polarisierten Abfühlparameter;
Entfernung oder Verschiebung der Zellen der abdekkenden Zellenpopulation aus einem zweiten Probenteil durch Mischen von Mikrosphären oder Mikrokügelchen damit die damit verbunden ein Reaktionsmittel aufweisen, welches für ein spezielles Molekül an den Zellen der abdeckenden Zellenpopulation spezifisch ist;
Hindurchgehen der Zellen des zweiten Probenteils, im wesentlichen eine zu jeder Zeit durch eine einen polarisierten Lichtstrahl aufweisende Abfühlzone, und Abfühlen und Zählen der einzelnen Zellen des zweiten Probenteils mit einem nicht polarisierten Lichtstreu-Abfühlparameter; und
Vergleichen der zwei Zählerstände zum Erhalt des Beitrags der interessierenden abgedeckten Zellen.

5. Verfahren nach Anspruch 4, wobei das Verschieben einer abgefühlten Charakteristik der Zellen in dem zweiten Probenteil vorgesehen ist, und wobei die Mikrosphären wesentlich kleiner als die Zellen sind.

6. Verfahren nach einem vorhergehenden Anspruch, wobei die Probe aus Vollblut besteht, und wobei die abdekkende Zellenpopulation eine WBC Population ist, und wobei die oder jede Gruppe von interessierenden Zellen eine Sub- oder Unter-Satzpopulation der WBC Population ist.

7. Verfahren nach Anspruch 3 und 6, wobei das Verschieben der abgefühlten Charakteristika jeder der WBC Subsatzpopulationen vorgesehen ist und zwar durch Mischen beider Mikrosphären mit einem dritten Probenteil, und Vergleichen der Ergebnisse der ersten, zweiten und dritten Probenteile, um den Prozentsatz des Überlappens der ersten und zweiten WBC Populationssub-Sätze zu bestimmen.

8. Verfahren nach Anspruch 6 oder 7, wobei jeder Subsatz aus den CD2, CD4 und CD8 WBC Populationssub-Sätzen ausgewählt ist.

9. Verfahren zum Erhalt einer Analyse einer WBC Population in einer Vollblutprobe, wobei folgendes vorgesehen ist:
Hindruchtreten der Zellen der WBC Population, im wesentlichen eine zu jeder Zeit, durch eine einen polarisierten Lichstrahl aufweisende Abfühlzone, und Abfühlen und Zählen der einzelnen Zellen mit einem nicht polarisierten Lichtstreu-Abfühlparameter;
Mischen eines Reaktionsmittels mit dem Probenmikrokügelchen, mit denen das Reaktionsmittel verbunden ist, welches für ein spezielles Molekül an den Zellen eines WBC Populationssubsatzes spezifisch ist und Entfernen der Mikrokügelchen mit den daran gebundenen Zellen aus der Probe;
Hindurchgehen der Zellen der verbleibenden WBC Population, und zwar im wesentlichen eine zu jeder Zeit, durch eine einen polarisierten Lichtstrahl aufweisende Abfühlzone, und Abfühlen und Zählen der einzelnen Zellen mit einem nicht polarisierten Lichtstreu-Abfühlparameter; und
Vergleichen der zwei Zählerstände zum Erhalt der verbleibenden und/oder der entfernten WBC Population.

10. Verfahren zum Erhalt einer Analyse einer WBC Population in einer Vollblutprobe, wobei folgendes vorgesehen ist:
Hindurchführen der Zellen der WBC Population, im wesentlichen eine zu jeder Zeit, durch eine Abfühlzone mit einem polarisierten Lichtstrahl, und Abfühlen und Zählen der einzelnen Zellen mit einem nicht polarisierten Abfühllichtstreuparamater;
Verschieben der abgefühlten Charakteristika der Zellen eines WBC Populationssubsatzes durch Mischung mit einer Probe von Mikrosphären, die damit verbunden ein Reaktionsmittel aufweisen, welches für ein spezifisches Molekül auf den Zellen des Subsatzes spezifisch ist, wobei die Mikrosphären wesentlich kleiner sind als die Zellen;
Hindurchleiten der Zellen, im wesentlichen eine zu jeder Zeit, durch eine Abfühlzone einschließlich der individuellen Zellen mit einem nicht polarisierten Lichtstreu-Abfühlparameter; und
Vergleichen der zwei Zählerstände zum Erhalt der verbleibenden und/oder verschobenen WBC Populationen.

11. Verfahren nach einem der Ansprüche 1 bis 10, wobei die Vollblutprobe eine rote Blutkörperchenpopulation (rote Blutzellenpopulation) aufweist, wobei zusätzlich das Entfernen der roten Blutkörperchenpopulation aus der Probe vorgesehen ist, und zwar ohne die relevanten Qualitäten und/oder Quantitäten der WBC Population signifikanterweise nachteilig zu beeinflussen.

12. Verfahren nach einem vorhergehenden Anspruch, wobei das oder jedes Reaktionsmittel ein monoklonaler Antikörper ist und das oder jedes spezifische Molekül ein Antigen ist.

13. Verfahren nach Anspruch 12, wobei die monoklonalen Antikörper neutrophil spezifisch sind und die interessierenden Zellen eine unreife WBC Population aufweisen bzw. sind.

14. Verfahren nach einem vorhergehenden Anspruch, wobei die Zellen zusätzlich mit einem elektronischen Abfühlparameter abgefühlt werden, oder mit mindestens zwei unterschiedlichen elektronischen Abfühlparametern, um mindestens eine zweidimensionale Charakterisierung der Zellen zu liefern.

## Revendications

1. Un procédé pour obtenir une analyse de cellules intéressantes dans un échantillon renfermant une population de cellules masquantes, comprenant ' les opérations consistant :
à déplacer les caractéristiques détectées des cellules intéressantes par rapport aux caractéristiques détectées des cellules de la population de cellules masquantes en mélangeant à une première fraction de l'échantillon des microsphères auxquelles est lié un réactif spécifique d'une molécule spécifique présente sur les cellules intéressantes, les microsphères étant sensiblement plus petites que lesdites cellules ;
à faire traverser aux cellules, sensiblement une par une, une zone de détection comportant un faisceau de lumière polarisée, et à détecter et compter les cellules individuelles de la fraction d'échantillon avec un paramètre de détection non polarisé de diffusion de lumière ; et
à utiliser le paramètre détecté pour caractériser les cellules et pour obtenir la contribution des cellules intéressantes.

2. Un procédé selon la revendication 1, qui comprend en outre la détection et le comptage de la première fraction d'échantillon, avant l'opération de déplacement, et la comparaison des totaux de comptage respectifs, pour obtenir le pourcentage de contribution.

3. Un procédé selon la revendication 1 ou la revendication 2, qui comprend en outre le déplacement des caractéristiques détectées d'un deuxième groupe de cellules intéressantes dans la première ou dans une deuxième fraction d'échantillon par mélange à celle-ci de microsphères auxquelles est lié un réactif spécifique d'une molécule spécifique présente sur le deuxième groupe de cellules intéressantes.

4. Un procédé pour obtenir une analyse de cellules intéressantes dans un échantillon renfermant une population de cellules masquantes, comprenant les opérations consistant :
à faire traverser aux cellules d'une première fraction d'échantillon, sensiblement une par une, une zone' de détection comportant un faisceau de lumière polarisée, et à détecter et compter les cellules individuelles de la première fraction avec un paramètre de détection non polarisé de diffusion de lumière ;
à éliminer ou à déplacer les cellules de la population de cellules masquantes d'une deuxième fraction d'échantillon en y mélangeant des microsphères auxquelles est lié un réactif qui est spécifique d'une molécule spécifique présente sur les cellules de la population de cellules masquantes ;
à faire traverser aux cellules de la deuxième fraction d'échantillon, sensiblement une par une, une zone de détection comportant un faisceau de lumière polarisée, et à détecter et compter les cellules individuelles de la deuxième fraction d'échantillon avec un paramètre de détection non polarisé de diffusion de lumière ; et
à comparer les deux totaux de comptage, pour obtenir la contribution des cellules masquantes intéressantes.

5. Un procédé selon la revendication 4, qui comprend le déplacement d'une caractéristique détectée des cellules de la deuxième fraction d'échantillon, les microsphères étant sensiblement plus petites que les cellules.

6. Un procédé selon l'une quelconque des revendications précédentes, dans lequel l'échantillon est un échantillon de sang entier, la population de cellules masquantes est une population de globules blancs sanguins, et le ou chaque groupe de cellules intéressantes est une sous-population de la population de globules blancs.

7. Un procédé selon les revendications 3 et 6 qui comprend le déplacement des caractéristiques détectées de chacune des sous-populations de globules blancs par mélange des deux sortes de microsphères avec une troisième fraction d'échantillon, et la comparaison des résultats des première, deuxième et troisième fractions, pour déterminer le pourcentage de recouvrement des première et deuxième sous-populations de globules blancs.

8. Un procédé selon la revendication 6 ou la revendication 7, dans lequel la ou chaque sous-population est choisie parmi les sous-populations de globules blancs CD2, CD4 et CD8.

9. Un procédé pour obtenir une analyse d'une population de globules blancs sanguins dans un échantillon de sang entier, comprenant les opérations consistant :
à faire traverser aux cellules de la population de globules blancs sanguins, sensiblement une par une, une zone de détection comportant un faisceau de lumière polarisée, et à détecter et compter les cellules individuelles avec un paramètre de détection non polarisé de diffusion de lumière ;
à mélanger à l'échantillon des microsphères auxquelles est lié un réactif spécifique d'une molécule spécifique portée par les cellules d'une sous-population de la population de globules blancs, et à éliminer de l'échantillon les microsphères auxquelles sont liées des cellules ;
à faire traverser aux cellules de la population de globules blancs restante, sensiblement une par une, une zone de détection comportant un faisceau de lumière polarisée, et à détecter et compter les cellules individuelles avec un paramètre de détection non polarisé de diffusion de lumière ; et
à comparer les deux totaux de comptage, pour obtenir la population de globules blancs restante et/ou éliminée.

10. Un procédé pour obtenir une analyse d'une population de globules blancs sanguins dans un échantillon de sang entier, comprenant les opérations consistant :
à faire traverser aux cellules de la population de globules blancs, sensiblement une par une, une zone de détection comportant un faisceau de lumière polarisée, et à détecter et compter les cellules individuelles avec un paramètre de détection non polarisé de diffusion de lumière ;
à déplacer les caractéristiques détectées des cellules d'une sous-population de globules blancs sanguins en mélangeant à l'échantillon des microsphères auxquelles est lié un réactif spécifique d'une molécule spécifique présente sur les cellules de la sous-population, les microsphères étant sensiblement plus petites que les cellules ;
à faire traverser aux cellules, sensiblement une par une, une zone de détection comportant les cellules individuelles avec un paramètre de détection non polarisé de diffusion de lumière ; et
à comparer les deux totaux de comptage, pour obtenir les populations de globules blancs restantes et/ou éliminées.

11. Un procédé selon l'une quelconque des revendications 6 à 10, dans lequel l'échantillon de sang entier comprend une population de globules rouges, et qui comprend en outre l'élimination de la population de globules rouges de l'échantillon sans nuire appréciablement aux qualités et/ou quantités caractéristiques de la population de globules blancs.

12. Un procédé selon l'une quelconque des revendications précédentes, dans lequel le ou chaque réactif est un anticorps monoclonal et la ou chaque molécule spécifique est un antigène.

13. Un procédé selon la revendication 12, dans lequel les anticorps monoclonaux sont spécifiques des neutrophiles et les cellules intéressantes comprennent une population de globules blancs sanguins immatures.

14. Un procédé selon l'une quelconque des revendications précédentes, dans lequel les cellules sont détectées en outre avec un paramètre de détection électronique, ou avec au moins deux paramètres de détection électronique, pour obtenir au moins une caractérisation bidimensionnelle des cellules.
